# EUROPÄISCHE PATENTSCHRIFT

(11) **EP 3 527 230 B1**
(45) Veröffentlichungstag und Bekanntmachung des Hinweises auf die Patenterteilung: **10.04.2024**
(21) Anmeldenummer: 18157711.5
(22) Anmeldetag: 20.02.2018
(51) Int. Cl.: A61L 2/08, A23L 3/26

(54) **VORRICHTUNG UND VERFAHREN ZUM PASTEURISIEREN UND/ODER STERILISIEREN VON PARTIKELFÖRMIGEM GUT**
DEVICE AND METHOD FOR PASTEURISING AND/OR STERILISING PARTICULATE MATERIAL
DISPOSITIFS ET PROCÉDÉ DE PASTEURISATION ET/OU DE STÉRILISATION DE PRODUITS PARTICULAIRES

(43) Veröffentlichungstag der Anmeldung: 21.08.2019
(73) Patentinhaber: Bühler AG, 9240 Uzwil (CH)
(72) Erfinder: CURRIE, Alasdair, London N7 8QB (GB); HERSCHE, Martin, 9230 Flawil (CH)
(74) Vertreter: Hepp Wenger Ryffel AG

(56) Entgegenhaltungen:
- EP-A1- 2 371 397
- EP-A1- 2 668 963
- EP-A1- 2 845 609
- EP-B1- 0 705 531
- EP-B1- 1 080 623
- WO-A1-91/11096
- WO-A1-03/017747
- WO-A1-2013/182500
- WO-A2-99/39750
- LIANZHONG D ET AL: "A study on chemical composition of spices irradiated by electron beam", RADIATION PHYSICS AND CHEMI, ELSEVIER, AMSTERDAM, NL, Bd. 52, Nr. 1-6, 1. Juni 1998 (1998-06-01) , Seiten 49-52, XP004492856, ISSN: 0969-806X, DOI: 10.1016/S0969-806X(98)00072-3

## Beschreibung

Die vorliegende Erfindung betrifft Vorrichtungen und Verfahren zum Pasteurisieren oder Sterilisieren von partikelförmigem Gut mit Hilfe eines Elektronenstrahls.

Als partikelförmig werden hier und im Folgenden unter anderem aus Körnern und/oder Flocken bestehende Güter bezeichnet, wobei die Partikel beispielsweise eine kugelförmige, plattenförmige oder kantige Form haben können. Es kann sich auch um gemahlene Partikel handeln. Durch die Pasteurisierung oder Sterilisierung können beispielsweise Mikroorganismen zumindest grösstenteils abgetötet oder unschädlich gemacht werden. Insbesondere kann eine Reduktion von schädigenden Mikroorganismen um mindestens eine, bevorzugt mindestens fünf, besonders bevorzugt mindestens sieben Grössenordnungen erreicht werden.

Eine gattungsgemässe Vorrichtung ist beispielsweise aus der EP 1 080 623 B1 bekannt. Diese Vorrichtung enthält Vibrationsförderer, mit denen Saatgut zu einem transparenten Vorhang vereinzelt werden kann. Dieser Vorhang wird dann durch ein Elektronenfeld geführt, das von einem Elektronenbeschleuniger erzeugt wird und beispielsweise eine Sterilisierung des Saatguts bewirken kann. Um das Saatgut von einem Austrittsfenster des Elektronenbeschleunigers fernzuhalten, wird ein Gitter eingesetzt.

Aus der US 5,801,387 A ist eine weitere gattungsgemässe Vorrichtung bekannt. In der dortigen erfindungsgemässen Ausführung wird ein partikelförmiges Gut mit einem Vibrationsförderer in einen horizontalen Luftstrom eindosiert und dann einem Elektronenstrahl ausgesetzt. Anschliessend wird mit Hilfe einer Vakuumpumpe und eines Filters eine Klassierung vollzogen.

Weiterhin offenbart die DE 10 2012 209 434 A1 eine Vorrichtung, die ein rieselfähiges Produkt mit Hilfe einer Vibrationsfördereinrichtung und einer rotierenden Bürstenwalze vereinzelt und in Rotationen versetzt. Anschliessend passieren die Partikel frei fallend ein Elektronenfeld.

In der EP 0 513 135 B1 ist eine Vorrichtung offenbart, mit der Saatgut mittels Zellradschleusen in einen vertikalen Fallschacht eingeleitet wird, wo es im senkrechten Fall von Elektronenstrahlen beaufschlagt wird.

Aus der EP 0 705 531 B1 ist eine weitere Vorrichtung bekannt, die das Saatgut mittels einer nicht näher beschriebenen Dosiereinrichtung in eine Prozesskammer eingeleitet wird, in der es senkrecht durch einen Elektronenstrahl fällt.

Weitere Vorrichtungen und Verfahren zum Pasteurisieren oder Sterilisieren von partikelförmigem Gut sind aus der internationalen Patentanmeldung WO 2018/036899 A1 der Anmelderin offenbart. Die Vorrichtungen enthalten mindestens eine Elektronenquelle zum Erzeugen eines Elektronenstrahls und eine Behandlungszone, in der das Gut mittels des Elektronenstrahls pasteurisierbar oder sterilisierbar ist. In einem ersten Aspekt enthält die Vorrichtung weiterhin eine erste Vibrationsfläche, welche zu Vibrationen anregbar ist, um das Gut zu fördern und zu vereinzeln, wobei die erste Vibrationsfläche eine Vielzahl von Rinnen aufweist, in denen das Gut förderbar und mittels deren es vereinzelbar ist. In einem zweiten Aspekt weist die Vorrichtung im Bereich der Behandlungszone einen Gutkanal auf, in dem das Gut mittels des Elektronenstrahls pasteurisierbar oder sterilisierbar ist, wobei die Vorrichtung mindestens einen von einem Fluid durchströmbaren Nebenkanal aufweist, welcher zumindest teilweise zwischen der Elektronenquelle und dem Gutkanal verläuft und vom Gutkanal fluidgetrennt ist. Weiterhin offenbart sind eine Kassette zum Einsetzen in eine Vorrichtung zum Pasteurisieren oder Sterilisieren von partikelförmigem Gut.

Bei der Behandlung des Guts in der Behandlungszone kann Strahlung entstehen, die für die Umgebung, insbesondere für das Bedienpersonal, schädlich sein kann. Zudem kann die Strahlung auch auf das Gut selbst einen negativen Einfluss haben, solange es in der Vorrichtung geführt wird, insbesondere abseits von der Behandlungszone.

Weiterhin offenbart das Dokument WO 2013/182500 A1 ein Verfahren zur Behandlung rieselfähiger Produkte aus vereinzelbaren Partikeln, vorzugsweise Saatgut, mit beschleunigten Elektronen. Bei dem Verfahren wird ein transparenter Produktstrom in einem Produktführungskanal durch das von mindestens einem Elektronenbeschleuniger erzeugte Elektronenfeld bei Unter- oder Überdruck unter Nutzung der Schwerkraft geführt. Die Führung des Produktstromes erfolgt mittels eines beschleunigten Gasstromes derart, dass dessen Bewegung in Betrag und Richtung der beschleunigten Bewegung entspricht, die die in ihm fallenden Partikel aufgrund der Erdbeschleunigung ausführen. Darüber hinaus wird eine Vorrichtung offenbart, die durch die Ausformung des Produktführungskanals die Beschleunigung des Gasstromes in der erforderlichen Weise realisiert.

Die WO 99/39750 A2 offenbart ein Verfahren zum Bestrahlen eines Gutes, insbesondere eines Materials, eines Abfalls, eines Bauteils, eines Lebensmittels, einer Flüssigkeit, eines Gases oder dgl., bei dem das Gut mit einem Fördersystem durch einen Strahl bewegter Teilchen aus zumindest einer Bestrahlungseinrichtung, insbesondere einem Elektronenbeschleuniger, in einer Bestrahlungskammer bewegt wird. Die zur Bestrahlung benötigten, aus einer Glühkathode austretenden Elektronen werden fokussiert, in einer Beschleunigereinheit mit Wellen einer bestimmten, vordefinierbaren Frequenz gepulst, treten mit einer bestimmten Frequenz aus der Elektronenaustrittsvorrichtung aus und werden auf das zu bestrahlende Gut gelenkt.

Offenbart in der EP 2 371 397 A1 ist eine Vorrichtung zum Sterilisieren von Behältnissen mit einer Transporteinrichtung, welche die Behältnisse entlang eines vorgegebenen Transportpfades transportiert, mit einer Vielzahl von Sterilisationseinrichtungen zum Sterilisieren wenigstens eines Bereiches der Innenwandung der Behältnisse, wobei die ersten Sterilisationseinrichtungen jeweils ein stangenartiges Sterilisationselement aufweisen, dessen Längsrichtung sich im Wesentlichen senkrecht zu dem Transportpfad der Behältnisse erstreckt, und welches durch eine Relativbewegung des Behältnisses gegenüber dem Stangenelement in das Innere der zu sterilisierenden Behältnisse einführbar ist, sowie eine Strahlungserzeugungseinrichtung, welche eine sterilisierende Strahlung erzeugt, wobei die Vorrichtung wenigstens eine zweite Sterilisationseinrichtung zur Sterilisation wenigstens einer Aussenwandung der Behältnisse aufweist, wobei die zweite Sterilisationseinrichtung eine Strahlungserzeugungseinrichtung aufweist, welche eine sterilisierende Strahlung erzeugt.

Zudem offenbart EP 2 845 609 A1 eine Vorrichtung zum Sterilisieren von Behältnissen mit einer Transporteinrichtung, welche die zu sterilisierenden Behältnisse entlang eines vorgegebenen Transportpfads (P), mit wenigstens einer Sterilsationseinrichtung (4, 6a, 6b), welche die Behältnisse während ihres Transports zu ihrer Sterilisation mit Ladungsträgern beaufschlagt, wobei diese Sterilisationseinrichtung (4, 6a, 6b) eine Erzeugungseinrichtung zum Erzeugen von Ladungsträgern aufweist, und mit einer Abschirmeinrichtung zum Abschirmen von entstehender Strahlung, welche wenigstens abschnittsweise den Transportpfades (P) der Behältnisse umgibt. Die Abschirmeinrichtung weist eine erste Wandung auf, welche seitlich neben dem Transportpfad der Behältnisse angeordnet ist, sowie eine zweite Wandung, welche unterhalb des Transportpfades der Behältnisse angeordnet ist, wobei die zweite Wandung gegenüber dem Transportpfad (P) der Behältnisse bewegbar ist.

Gegenstand der WO 91/11096 A1 sind ein Verfahren und eine Vorrichtung zur Elektronenstrahlbehandlung von Schüttgut, bei der das Schüttgut mittels Kompressionsstufen, die evakuiert werden können, in einen kontinuierlichen Strom eingespeist wird. Das Schüttgut wird dispergiert und in eine dosierte Bestrahlungskammer geleitet, die in dieser Kammer im freien Fall durch ein Bestrahlungsfeld geleitet und durch ein Kompressionsstufensystem aus der Bestrahlungskammer abgegeben wird. Hierdurch soll eine besonders strahlende Elektronenstrahlbehandlung von biologischen Materialien ermöglicht werden, beispielweise bei der Bekämpfung von Schadorganismen in Samen.

Ausserdem ist in der WO 03/017747 A1 ein Verfahren zum Bestrahlen von Schüttgut offenbart, insbesondere zur Behandlung von Nahrungsmitteln wie Getreide, das dadurch gekennzeichnet ist, dass im Falle eines Versagens der Bestrahlung das unbehandelte Schüttgut von dem behandelten Material abgetrennt und anschliessend zurück in Richtung Beginn des Prozesses transportiert wird, um behandelt zu werden. Hierdurch werde es ermöglicht, eine erneute Behandlung grosser Materialmengen zu vermeiden und stellt eine klare Trennung zwischen kontaminierten und nicht kontaminierten Teilen der Anlage bereit.

Es ist eine Aufgabe der vorliegenden Erfindung, die aus dem Stand der Technik bekannten Nachteile zu überwinden. Insbesondere sollen Vorrichtungen und Verfahren bereitgestellt werden, die die Auswirkungen der bei der Behandlung des Guts entstehenden Strahlung möglichst unterdrücken. Unter anderem sollen das Gut abseits von der Behandlungszone und auch die Umgebung der Vorrichtung, insbesondere deren Bediener, möglichst vor der Strahlung geschützt werden.

Die erfindungsgemässe Vorrichtung zum Pasteurisieren oder Sterilisieren von partikelförmigem Gut enthält ein äusseres Gehäuse, einen Guteinlauf, einen Gutauslauf, einen Gutführungskanal, in dem das Gut vom Guteinlauf entlang einer Gutführungsrichtung durch die Vorrichtung hindurch zum Gutauslauf führbar ist, mindestens eine innerhalb des Gehäuses angeordnete Elektronenquelle zum Erzeugen eines Elektronenstrahls sowie eine im Gutführungskanal angeordnete Behandlungszone, in der das Gut insbesondere frei fallend mittels des Elektronenstrahls pasteurisierbar oder sterilisierbar ist. Das Gut wird als "frei fallend" bezeichnet, wenn die Flugbahnen der einzelnen Partikel des Guts allein durch ihre Geschwindigkeit, die auf sie einwirkende Schwerkraft und gegebenenfalls ein Prozessgas, von dem das Gut umgeben ist, bestimmt werden. Insbesondere rutschen frei fallende Partikel des Guts nicht auf einer Fläche durch die Behandlungszone. Das Prozessgas kann beispielsweise Luft sein. Es ist jedoch auch denkbar, dass als Prozessgas ein Gas eingesetzt wird, welches eine Ozonbildung verhindert, wie beispielsweise Stickstoff.

Erfindungsgemäss weist die Vorrichtung mindestens eine innerhalb des äusseren Gehäuses angeordnete und den Gutführungskanal insbesondere entlang der Gutführungsrichtung umschliessende innere Abschirmung mit mindestens einem Abschirmelement zum Abschirmen der bei der Behandlung entstehenden Strahlung auf.

Als innere Abschirmung wird im Rahmen der vorliegenden Erfindung eine weitere, innerhalb des äusseren Gehäuses angeordnete Abschirmung verstanden, deren Abschirmelemente höchstens teilweise oder gar nicht zum äusseren Gehäuse gehören. Vorteilhafterweise bildet das äussere Gehäuse der Vorrichtung ebenfalls eine Abschirmung für die bei der Behandlung entstehende Strahlung.

Aufgrund der inneren Abschirmung werden die Risiken aufgrund der bei der Behandlung entstehenden Strahlung deutlich weiter reduziert, als dies mit nur dem äusseren Gehäuse möglich wäre. Hierdurch kann das Gut auch innerhalb der Vorrichtung, nämlich abseits von der Behandlungszone, vor der potentiell schädlichen Strahlung geschützt werden. Zudem kann die Umgebung der Vorrichtung, insbesondere deren Bediener, noch wirkungsvoller vor möglichen Risiken der Strahlung geschützt werden.

Bevorzugt weist der Gutführungskanal mindestens einen Kanalabschnitt auf, in dem ihn die Abschirmung labyrinthartig umschliesst. Unter einer labyrinthartigen Umschliessung durch die Abschirmung wird hier und im Folgenden verstanden, dass die Abschirmelemente derart ausgebildet und angeordnet sind, dass sie eine geradlinige Ausbreitung der in der Behandlungszone entstehenden Strahlung aus der Behandlungszone hinaus in die Umgebung der Vorrichtung, also in den Raumbereich ausserhalb des äusseren Gehäuses der Vorrichtung, verhindern können. Hierdurch kann die Abschirmwirkung nochmals verbessert werden.

Bevorzugt ist es, wenn der Gutführungskanal einen Guteinlaufkanal enthält, in dem das Gut vom Guteinlauf zur Behandlungszone führbar ist, wobei mindestens eine innere Abschirmung den Guteinlaufkanal labyrinthartig umschliesst. Eine labyrinthartige Umschliessung wird dabei wie oben verstanden. Hierdurch kann verhindert werden, dass die in der Behandlungszone entstehende Strahlung bereits auf das Gut trifft, bevor es die Behandlungszone erreicht hat.

Ebenfalls bevorzugt ist es, wenn der Gutführungskanal einen Gutauslaufkanal enthält, in dem das Gut von der Behandlungszone zum Gutauslauf führbar ist, wobei mindestens eine innere Abschirmung den Gutauslaufkanal labyrinthartig umschliesst. Dies verhindert, dass die in der Behandlungszone entstehende Strahlung auch noch dann auf das Gut trifft, wenn es die Behandlungszone bereits verlassen hat.

Das Gehäuse weist weiterhin erfindungsgemäss mindestens eine Öffnung auf, welche mittels mindestens eines ihr zugeordneten Verschlusselements verschliessbar ist, wobei das Verschlusselement an einer Innenseite mindestens eines der Abschirmelemente enthält. In einer Schliessposition des Verschlusselements verschliesst dieses die ihm zugeordnete Öffnung. Das Verschlusselement kann beispielsweise eine Tür sein. Dies ermöglicht es, dass in der Schliessposition des Verschlusselements auch im Bereich der Öffnung das Risiko des Austritts von Strahlung aus der Vorrichtung reduziert wird. Bei geöffnetem Verschlusselement sind viele der Komponenten der Vorrichtung zugänglich. Beispielsweise kann eine weiter unten noch beschriebene Kassette einfach zugänglich sein, die eine zwischen der Elektronenquelle und der Behandlungszone angeordnete Schutzfolie hält. Die vereinfachte Zugänglichkeit erleichtert bei Bedarf die Reinigung und den Austausch der Schutzfolie.

Es ist zweckmässig, wenn die Abschirmung ein statisches erstes Abschirmelement enthält und das Abschirmelement des Verschlusselements ein zweites Abschirmelement bildet, wobei das erste Abschirmelement und das zweite Abschirmelement in der Schliessposition des Verschlusselements den Gutführungskanal gemeinsam insbesondere labyrinthartig umschliessen.

Die Abschirmelemente können beispielsweise Blei enthalten oder daraus bestehen. In einer bevorzugten Ausführungsform haben die Abschirmelemente einen mehrlagigen Aufbau mit mindestens einer inneren Lage aus Blei und zwei äusseren Lagen aus einem anderen Material. Die äusseren Lagen sollten bevorzugt aus einem Material bestehen, welches in Kontakt mit dem behandelten Gut, insbesondere einem Lebensmittel, gelangen darf. Beispielsweise können die äusseren Lagen aus rostfreiem Stahl oder einem Kunststoff bestehen.

Weiterhin ist es von Vorteil, wenn der Gutführungskanal durch Wandungselemente begrenzt ist, von denen mindestens eines und bevorzugt sämtliche beweglich oder sogar lösbar direkt oder indirekt mit dem Gehäuse verbunden oder verbindbar sind. Dies vereinfacht den Austausch der lösbaren Wandungselemente, beispielsweise wenn diese defekt sind oder gereinigt werden müssen oder wenn für verschiedene mit der Vorrichtung behandelte Güter verschiedene Wandungselemente benötigt werden. Als Wandungselemente des Gutführungskanals werden unter anderem auch die nachfolgend noch beschriebenen, optionalen Verschlusselemente, Vibrationsflächen Rutschflächen und Schutzfolien verstanden.

Die mindestens eine Elektronenquelle kann an sich bekannt sein. Die Vorrichtung kann eine oder mehrere Elektronenquellen enthalten. Sind mehrere Elektronenquellen vorhanden, so können diese einander gegenüberliegend oder bezüglich der Strömungsrichtung des Guts nacheinander angeordnet sein.

Weiterhin ist es auch denkbar und liegt im Rahmen der Erfindung, dass die Vorrichtung mehrere Behandlungszonen aufweist. Auf diese Weise kann eine noch effektivere Pasteurisierung oder Sterilisierung erreicht werden. Alternativ kann das Gut mehrmals durch ein und dieselbe Behandlungszone geführt werden.

Die Vorrichtung kann auch eines oder mehrere der Merkmale aufweisen, die in der eingangs bereits erwähnten internationalen Patentanmeldung WO 2018/036899 A1 der Anmelderin offenbart sind:
I. Vorrichtung zum Pasteurisieren oder Sterilisieren von partikelförmigem Gut, enthaltend
   - eine bevorzugt im Wesentlichen horizontal ausgerichtete erste Vibrationsfläche, welche zu Vibrationen anregbar ist, um das Gut zu fördern und zu vereinzeln,
   - mindestens eine Elektronenquelle zum Erzeugen eines Elektronenstrahls,
   - eine stromabwärts von der ersten Vibrationsfläche angeordnete Behandlungszone, in der das Gut insbesondere frei fallend mittels des Elektronenstrahls pasteurisierbar oder sterilisierbar ist,
   wobei die erste Vibrationsfläche eine Vielzahl von Rinnen aufweist, in denen das Gut förderbar und mittels deren es vereinzelbar ist.
II. Vorrichtung gemäss Merkmalskombination I,
   wobei die Vorrichtung stromabwärts von der ersten Vibrationsfläche und stromaufwärts von der Behandlungszone eine geneigte Rutschfläche aufweist, welche derart ausgebildet und angeordnet ist, dass das Gut darauf in Richtung der Behandlungszone rutschen kann.
III. Vorrichtung gemäss Merkmalskombination II,
   wobei die Rutschfläche mindestens eine Rinne, bevorzugt eine Vielzahl von Rinnen aufweist, welche derart ausgebildet und angeordnet ist/sind, dass das Gut darin rutschen und vereinzelt werden kann.
IV. Vorrichtung gemäss einem der Merkmalskombinationen II und III,
   wobei die Rutschfläche bezüglich einer Horizontalen unter einem Winkel nach unten geneigt ist, der im Bereich von 45° bis 85°, bevorzugt von 55° bis 75°, besonders bevorzugt von 60° bis 70° liegt.
V. Vorrichtung gemäss einer der vorangehenden Merkmalskombinationen,
   wobei die Vorrichtung stromabwärts von der ersten Vibrationsfläche und stromaufwärts von der Behandlungszone, insbesondere stromaufwärts von der Rutschfläche, eine Umlenkfläche aufweist, welche derart ausgebildet und angeordnet ist, dass das Gut darauf umgelenkt und von der ersten Vibrationsfläche zur Rutschfläche und/oder in Richtung der Behandlungszone rutschen kann.
VI. Vorrichtung gemäss Merkmalskombination V,
   wobei die Umlenkfläche mindestens eine Rinne, bevorzugt eine Vielzahl von Rinnen aufweist, welche derart ausgebildet und angeordnet ist/sind, dass das Gut darin rutschen kann.
VII. Vorrichtung gemäss einer der vorangehenden Merkmalskombinationen,
   wobei die Vorrichtung stromaufwärts von der ersten Vibrationsfläche eine im Wesentlichen ebene und bevorzugt im Wesentlichen horizontal ausgerichtete zweite Vibrationsfläche aufweist, welche zu Vibrationen anregbar ist.
VIII. Vorrichtung zum Pasteurisieren oder Sterilisieren von partikelförmigem Gut, enthaltend
   - mindestens eine Elektronenquelle zum Erzeugen eines Elektronenstrahls,
   - eine Behandlungszone, in der das Gut insbesondere frei fallend mittels des Elektronenstrahls pasteurisierbar oder sterilisierbar ist,

   insbesondere Vorrichtung gemäss einer der vorangehenden Merkmalskombinationen,
   wobei die Vorrichtung im Bereich der Behandlungszone einen Gutkanal aufweist, in dem das Gut mittels des Elektronenstrahls pasteurisierbar oder sterilisierbar ist,
   wobei die Vorrichtung mindestens einen von einem Fluid durchströmbaren Nebenkanal aufweist, welcher zumindest teilweise zwischen der Elektronenquelle und dem Gutkanal verläuft und vom Gutkanal fluidgetrennt ist.
IX. Vorrichtung gemäss Merkmalskombination XIII,
   wobei zwischen der Elektronenquelle und dem Gutkanal eine für den Elektronenstrahl zumindest teilweise durchlässige und insbesondere aus einem Metall, bevorzugt aus Titan, bestehende, Schutzfolie angeordnet ist.
X. Vorrichtung gemäss Merkmalskombination IX,
   wobei die Schutzfolie den Gutkanal vom Nebenkanal trennt.
XI. Vorrichtung gemäss einem der Merkmalskombinationen IX und X,
   wobei der Nebenkanal zumindest teilweise zwischen der Elektronenquelle und der Schutzfolie angeordnet ist.
XII. Vorrichtung gemäss einem der Merkmalskombinationen IX bis XI,
   wobei die Vorrichtung eine Kassettenaufnahme zum Aufnehmen einer Kassette aufweist, wobei die Kassette zumindest teilweise den Gutkanal und den mindestens einen Nebenkanal begrenzt und eine Folienaufnahme zur Aufnahme der Schutzfolie enthält, und die Elektronenquelle derart beweglich, insbesondere schwenkbar und/oder verschiebbar, relativ zur Kassettenaufnahme angeordnet ist, dass sie von der Kassette weg bewegbar ist.
XIII. Vorrichtung gemäss Merkmalskombination XII,
   wobei eine Kassette in der Kassettenaufnahme eingesetzt ist, die zumindest teilweise den Gutkanal und den mindestens einen Nebenkanal begrenzt und eine Folienaufnahme enthält, von der die Schutzfolie aufgenommen ist.
XIV. Vorrichtung gemäss einer der vorangehenden Merkmalskombinationen,
   wobei die Vorrichtung eine Absaugeinrichtung zum Absaugen von das Gut umgebendem Prozessgas stromabwärts von der Behandlungszone enthält.
XV. Vorrichtung gemäss einer der vorangehenden Merkmalskombinationen,
   wobei die Vorrichtung stromabwärts von der Behandlungszone eine Sortiereinrichtung aufweist, welche eine Messeinheit und eine Ausstosseinheit enthält, welche derart ausgebildet sind, dass mittels der Ausstosseinheit einzelne Partikel des Guts anhand mindestens einer mittels der Messeinheit gemessenen Eigenschaft der Partikel ausstossbar sind.
XVI. Vorrichtung gemäss einer der vorangehenden Merkmalskombinationen,
   wobei die Vorrichtung mindestens eine stromabwärts von der Behandlungszone angeordnete Gasaustrittsöffnung zum Einblasen eines Reinigungsgases auf das Gut aufweist.

Die oben und auch nachfolgend verwendeten Begriffe "stromabwärts" und "stromaufwärts" beziehen sich auf die Gutführungsrichtung, also die Strömungsrichtung des partikelförmigen Guts bei bestimmungsgemässer Bedienung der Vorrichtung. Folglich wird eine erste Einheit als stromabwärts von einer zweiten Einheit bezeichnet, wenn sie bei bestimmungsgemässer Bedienung der Vorrichtung vom Gut nach der zweiten Einheit passiert wird. Analog wird eine erste Einheit als stromaufwärts von einer zweiten Einheit bezeichnet, wenn sie bei bestimmungsgemässer Bedienung der Vorrichtung vom Gut vor der zweiten Einheit passiert wird.

In einem weiteren Aspekt betrifft die Erfindung auch ein Verfahren zum Pasteurisieren oder Sterilisieren von partikelförmigem Gut. Dieses Verfahren enthält die folgenden Schritte:
a) Erzeugen eines Elektronenstrahls,
b) Pasteurisieren oder Sterilisieren des insbesondere frei fallenden Guts mittels des Elektronenstrahls in einer Behandlungszone.

Für viele Güter, insbesondere für eine Vielzahl von Gewürzen, hat es sich als vorteilhaft erwiesen, wenn sich das Gut mit einer Geschwindigkeit durch die Behandlungszone bewegt, die im Bereich von 1 m/s bis 5 m/s, bevorzugt von 2 m/s bis 4 m/s, besonders bevorzugt von 2 m/s bis 3 m/s liegt. Diese Geschwindigkeit kann beispielsweise durch die Länge und den Neigungswinkel einer wie oben erwähnten, stromaufwärts angeordneten Rutschfläche eingestellt werden. Je höher die Geschwindigkeit des Guts ist, desto grösser ist der erreichbare Durchsatz. Beim freien Fall ist die Geschwindigkeit unabhängig vom Durchsatz, so dass beispielsweise Durchsätze im Bereich 100 kg/h bis 1000 kg/h bei der gleichen Geschwindigkeit erreicht werden können. Der Durchsatz kann von der Vibration einer Vibrationsfläche(n) und der Dimensionen und Orientierungen einer allfälligen Umlenk- und Rutschfläche abhängen. Zudem sinkt mit steigender Geschwindigkeit des Guts die Wahrscheinlichkeit von Kollisionen der Partikel mit der Elektronenquelle oder einer oben bereits erwähnten Schutzfolie. Andererseits dürfen die Geschwindigkeiten aber auch nicht zu gross gewählt werden, damit das Gut ausreichend lange im Elektronenstrahl verbleibt, um pasteurisiert oder sterilisiert zu werden.

Die Elektronen des Elektronenstrahls weisen bevorzugt eine Energie auf, die im Bereich von 80 keV bis 300 keV, bevorzugt von 140 keV bis 280 keV, besonders bevorzugt von 180 keV bis 260 keV liegt. Geringere Elektronenenergien würden keine ausreichende Pasteurisierung oder Sterilisierung erzeugen. Durch höhere Elektronenenergien liessen sich keine wesentlich höheren Grade der Pasteurisierung oder Sterilisierung erreichen.

Vorteilhafterweise wird das Gut dem Elektronenstrahl für eine Behandlungszeit ausgesetzt, die im Bereich von 5 ms bis 25 ms liegt. Denn für eine ausreichende Pasteurisierung oder Sterilisierung ist eine gewisse minimale Behandlungszeit nötig. Zu lange Behandlungszeiten haben keinen nennenswert erhöhten Grad der Pasteurisierung oder Sterilisierung gezeigt und würden zudem den Durchsatz verringern.

Ebenfalls mit Vorteil wird das Gut mittels des Elektronenstrahls einer Strahlendosis ausgesetzt, die im Bereich von 1 kGy bis 45 kGy, bevorzugt von 8 kGy bis 30kGy, besonders bevorzugt von 10 kGy bis 16 kGy liegt.

Die Elektronenstromdichte in der Behandlungszone liegt bevorzugt im Bereich von 10¹⁵ s⁻¹·cm⁻² bis 2,77·10¹⁵ s⁻¹·cm⁻².

Das Verfahren kann weiterhin eines oder mehrere der Merkmale aufweisen, die in der eingangs bereits erwähnten internationalen Patentanmeldung WO 2018/036899 A1 der Anmelderin offenbart sind:
XVII. Verfahren zum Pasteurisieren oder Sterilisieren von partikelförmigem Gut, insbesondere mit einer wie oben offenbarten Vorrichtung, enthaltend die folgenden Schritte:
   a) Fördern und Vereinzeln des Guts mittels einer bevorzugt im Wesentlichen horizontal ausgerichteten ersten Vibrationsfläche, welche zu Vibrationen angeregt wird und eine Vielzahl von Rinnen aufweist, in denen das Gut gefördert und mittels deren es vereinzelt wird,
   b) Erzeugen eines Elektronenstrahls,
   c) Pasteurisieren oder Sterilisieren des insbesondere frei fallenden Guts mittels des Elektronenstrahls in einer Behandlungszone.
XVIII. Verfahren zum Pasteurisieren oder Sterilisieren von partikelförmigem Gut, insbesondere mit einer wie oben offenbarten Vorrichtung, enthaltend die folgenden Schritte:
   b) Erzeugen eines Elektronenstrahls,
   c) Pasteurisieren oder Sterilisieren des insbesondere frei fallenden Guts mittels des Elektronenstrahls in einer Behandlungszone,

   insbesondere Verfahren gemäss Merkmalskombination XVII,
   wobei das Gut im Bereich der Behandlungszone durch einen Gutkanal strömt, in dem das Gut mittels des Elektronenstrahls pasteurisiert oder sterilisiert wird, dadurch gekennzeichnet, dass
   ein Fluid durch mindestens einen Nebenkanal strömt, welcher zumindest teilweise zwischen der Elektronenquelle und dem Gutkanal verläuft und vom Gutkanal fluidgetrennt ist.
XIX. Verfahren gemäss Merkmalskombination XVIII,
   wobei die Schutzfolie den Gutkanal vom Nebenkanal trennt.
XX. Verfahren gemäss einer der Merkmalskombinationen XVIII und XIX,
   wobei der Nebenkanal zumindest teilweise zwischen der Elektronenquelle und der Schutzfolie angeordnet ist.
XXI. Verfahren gemäss einer der Merkmalskombinationen XVII bis XX,
   wobei das Gut umgebendes Prozessgas nach der Pasteurisierung oder Sterilisierung abgesaugt wird, insbesondere mit einer Absauggeschwindigkeit, die das 1- bis 3-fache, bevorzugt das 1- bis 1,5-fache der Geschwindigkeit des Guts während der Pasteurisierung oder Sterilisierung beträgt.

Bei dem Gut kann es sich um ein Lebensmittel handeln, wie beispielsweise Getreide wie etwa Soja, Frühstückscerealien, Snacks, Nüsse wie etwa getrocknete Kokosnüsse, Mandeln, Erdnussbutter, Kakaobohnen, Schokolade, Schokoladenflüssigkeit, Schokoladenpulver, Schokoladenchips, Kakaoprodukte, Hülsenfrüchte, Kaffee, Samen wie etwa Kürbissamen, Gewürze (wie beispielsweise Kurkuma, insbesondere in Scheiben), Teemischungen, getrocknete Früchte, Pistazien, trockene Proteinprodukte, Bäckereiprodukte, Zucker, Kartoffelprodukte, Teigwaren, Babynahrung, getrocknete Eiprodukte, Sojaprodukte wie beispielsweise Sojabohnen, Verdickungsmittel, Hefen, Hefeextrakte, Gelatine oder Enzyme handeln.

Alternativ kann das Gut auch ein Tiernahrungsmittel sein, wie beispielsweise Pellets, Futter für Wiederkäuer, Geflügel, Wassertiere (insbesondere Fische) oder Haustiere, oder Mischfutter.

Es ist jedoch ebenso denkbar und liegt im Rahmen der Erfindung, dass das Gut beispielsweise ein Kunststoff wie etwa PET ist, beispielsweise in Form von Flocken oder Pellets.

Im Folgenden wird die Erfindung anhand eines Ausführungsbeispiels und mehrerer Zeichnungen näher erläutert. Dabei zeigen
- Figur 1:: eine perspektivische Ansicht einer erfindungsgemässen Vorrichtung;
- Figur 2:: eine Vorderansicht der Vorrichtung mit geöffneten Türen;
- Figur 3:: eine Rückansicht der Vorrichtung mit geöffneten Türen.

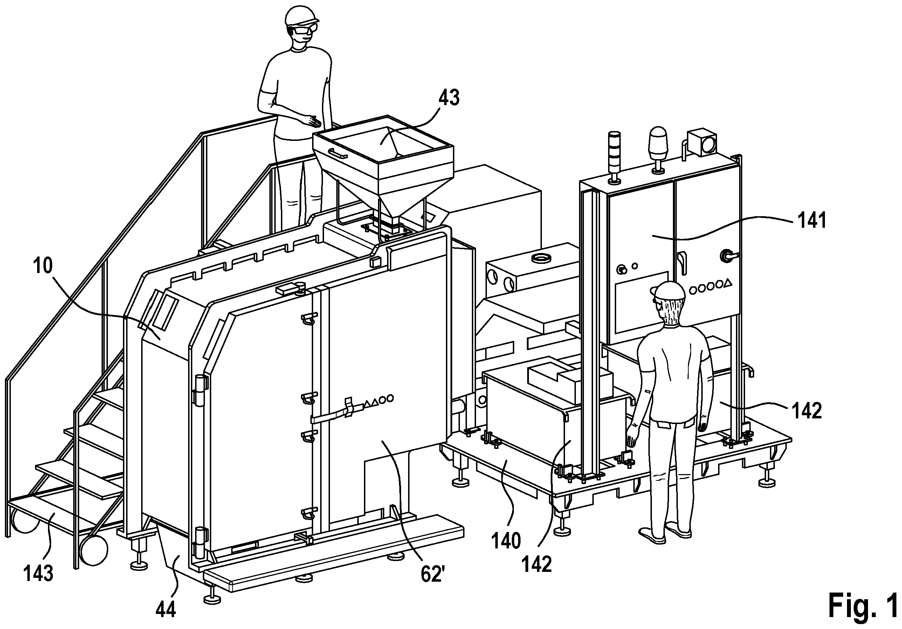

Die in den Figuren 1 bis 3 dargestellte Vorrichtung 10 ist zum Pasteurisieren oder Sterilisieren von partikelförmigem Gut vorgesehen, wie etwa einem Gewürz, Sesam, Mandeln oder geschälten Pistazien. Die Vorrichtung 10 enthält ein äusseres Gehäuse 40, einen Guteinlauf 43, einen Gutauslauf 44 und einen Gutführungskanal 41, in dem das Gut vom Guteinlauf 43 entlang einer Gutführungsrichtung R durch die Vorrichtung 10 hindurch zum Gutauslauf 44 führbar ist. Ausserhalb des äusseren Gehäuses 40 der Vorrichtung 10 befinden sich ein Elektrogestell 140 mit einem Schaltschrank 141 und zwei Generatoren 142 für die Versorgung der nachfolgend beschriebenen Elektronenquellen 20 und ein Podest 143.

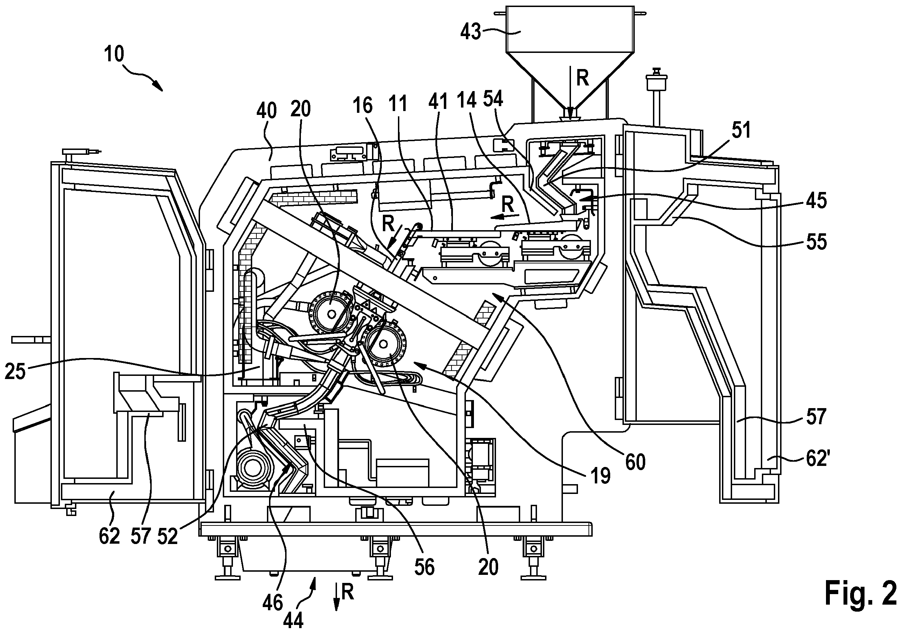

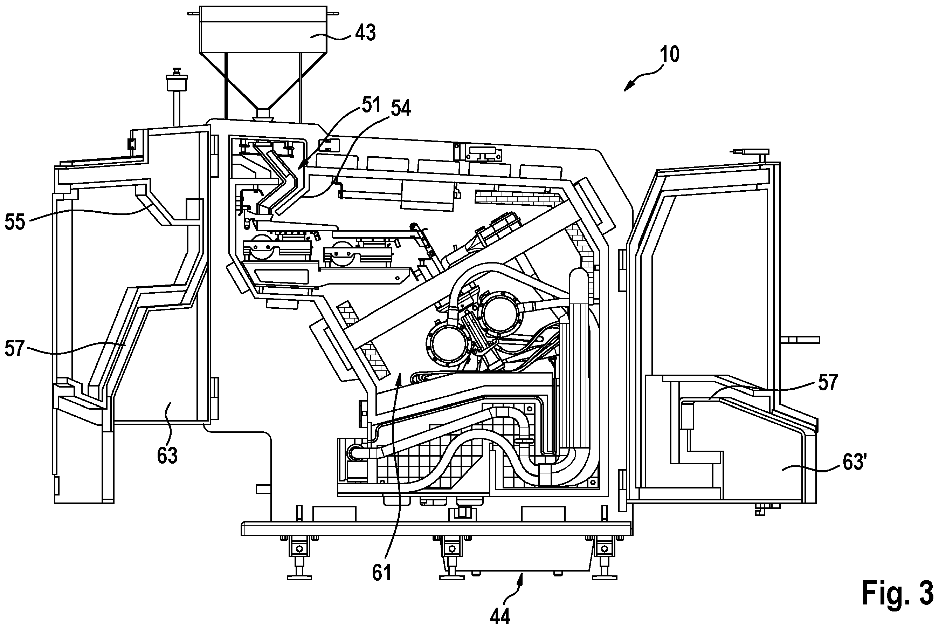

Die Vorrichtung 10 enthält gemäss den Figuren 2 und 3 weiterhin eine hier nicht dargestellte Dosiereinrichtung, mit der das Gut auf eine erste zu Schwingungen anregbare Vibrationsfläche 14 dosiert werden kann. Mit Hilfe dieser ersten Vibrationsfläche 14 kann der Durchsatz des Guts kontrolliert werden, und es kann auch bereits eine Vorvereinzelung stattfinden. Stromabwärts von der ersten Vibrationsfläche 14 enthält die Vorrichtung 10 eine zweite zu Schwingungen anregbare Vibrationsfläche 11. Hierdurch kann das Gut weiter stromabwärts gefördert und vereinzelt werden. Stromabwärts von der Vibrationsfläche 11 befindet sich eine geneigte Rutschfläche 16. Noch weiter stromabwärts enthält die Vorrichtung 10 eine Behandlungszone 19. Dort wird das Gut frei fallend mittels eines Elektronenstrahls pasteurisiert oder sterilisiert, der von zwei einander gegenüberliegenden Elektronenquellen 20 erzeugt wird. Die Vorrichtung 10 enthält weiterhin eine Absaugeinrichtung 25, mit dem Prozessgas, welches das Gut umgibt, stromabwärts von der Behandlungszone 19 abgesaugt werden kann. Die Vibrationsflächen 14 und 11 sowie die Rutschfläche 16 bilden einige der Wandungselemente, die den Gutführungskanal 41 begrenzen.

Gemäss der vorliegenden Erfindung weist die Vorrichtung 10 mehrere innerhalb des äusseren Gehäuses 40 angeordnete und den Gutführungskanal 41 entlang der Gutführungsrichtung R umschliessende innere Abschirmungen 51, 52 mit Abschirmelementen 54, 55, 56, 57 auf, die zum Abschirmen der bei der Behandlung in der Behandlungszone 19 entstehenden Strahlung dienen und beispielsweise aus Blei bestehen können.

Der Gutführungskanal 41 enthält zwei Kanalabschnitte 45, 46, in denen ihn die inneren Abschirmungen 51, 52 labyrinthartig umschliessen, unter anderem einen Guteinlaufkanal 45, in dem das Gut vom Guteinlauf 43 zur Behandlungszone 19 führbar ist, und einen Gutauslaufkanal 46, in dem das Gut von der Behandlungszone 19 zum Gutauslauf 44 führbar ist. Die den Guteinlaufkanal 45 umschliessende innere Abschirmung 51 verfügt über Abschirmelemente 54 und 55 und die den Gutauslaufkanal 46 umschliessende innere Abschirmung 52 über Abschirmelemente 56 und 57, wie nachfolgend noch näher erläutert wird.

Das äussere Gehäuse 40 der Vorrichtung 10 verfügt über mehrere Öffnungen, nämlich unter anderem über eine in Figur 2 dargestellte vordere Öffnung 60 und eine in Figur 3 gezeigte hintere Öffnung 61. Die vordere Öffnung 60 ist mittels einer ersten Tür 62 und einer zweiten Tür 62' in einer Schliessposition verschliessbar, und die hintere Öffnung 61 ist mittels einer dritten Tür 63 und einer vierten Tür 63' in einer Schliessposition verschliessbar. An den Innenseiten 64 enthalten die Türen 62, 62', 63, 63' die Abschirmelemente 55 und 57. Die Vorrichtung 10 enthält ferner statische Abschirmelemente 54 und 56. In der in Figur 1 gezeigten Schliessposition der Türen 62, 62', 63, 63' umschliessen die Abschirmelemente 54, 55, 56, 57 gemeinsam den Gutführungskanal 41 labyrinthartig.

Zum Pasteurisieren oder Sterilisieren von partikelförmigem Gut mit Hilfe dieser Vorrichtung 10 werden die folgenden Schritte durchgeführt:
Mittels der ersten Vibrationsfläche 14 wird der Durchsatz des Guts kontrolliert, und es findet eine Vorvereinzelung statt. Auf der zweiten Vibrationsfläche 11 wird das Gut weiter gefördert und vereinzelt. Mittels der Elektronenquellen 20 wird ein Elektronenstrahl erzeugt. Anschliessend erfolgt ein Pasteurisieren oder Sterilisieren des frei fallenden Guts mittels des Elektronenstrahls in der Behandlungszone 19.

Das Gut bewegt sich im Falle von Gewürzen vorteilhaft mit einer Geschwindigkeit von 2,5 m/s durch die Behandlungszone 19. Diese Geschwindigkeit kann durch die Länge und den Neigungswinkel der Rutschfläche 16 eingestellt werden. Die Elektronen des Elektronenstrahls weisen eine Energie auf, die im Bereich von 80 keV bis 300 keV liegt, beispielsweise bei 250 keV. In der Behandlungszone 19 weist der Elektronenstrahl eine mittlere Stromdichte auf, die im Bereich von 10¹⁵ s⁻¹·cm⁻² bis 2,77·10¹⁵ s⁻¹·cm⁻² liegt. Das Gut wird dem Elektronenstrahl für eine Behandlungszeit ausgesetzt, die im Bereich von 5 ms bis 25 ms liegen und beispielsweise 15 ms betragen kann. Hierdurch wird das Gut einer Strahlendosis ausgesetzt, die im Bereich von 1 kGy bis 45 kGy liegen und beispielsweise 12 kGy betragen kann. Das das Gut umgebende Prozessgas wird nach der Pasteurisierung oder Sterilisierung in der Behandlungszone 19 mittels der Absaugeinrichtung 25 abgesaugt, und zwar mit einer bevorzugten Absauggeschwindigkeit, die das 1- bis 1,5-fache Geschwindigkeit des Guts während der Pasteurisierung oder Sterilisierung beträgt.

Aufgrund der inneren Abschirmungen 51, 52 werden die Risiken aufgrund der bei der Behandlung in der Behandlungszone 19 entstehenden Strahlung deutlich weiter reduziert, als dies mit nur dem äusseren Gehäuse 40 möglich wäre. Hierdurch kann das Gut auch innerhalb der Vorrichtung 10, nämlich abseits von der Behandlungszone 19, vor der potentiell schädlichen Strahlung geschützt werden. Zudem kann die Umgebung der Vorrichtung 10, insbesondere deren Bediener, noch wirkungsvoller vor möglichen Risiken der Strahlung geschützt werden. Bei geöffneten Türen 62, 62', 63, 63' sind viele der Komponenten der Vorrichtung zugänglich - insbesondere eine im Bereich der Behandlungszone 19 angeordnete Kassette 24, die zwei aus Titan bestehende Schutzfolien 23 hält, durch die der Elektronenstrahl durchtritt.

## Patentansprüche

1. Vorrichtung (10) zum Pasteurisieren oder Sterilisieren von partikelförmigem Gut, enthaltend
- ein äusseres Gehäuse (40),
- einen Guteinlauf (43),
- einen Gutauslauf (44),
- einen Gutführungskanal (41), in dem das Gut vom Guteinlauf (43) entlang einer Gutführungsrichtung (R) durch die Vorrichtung (10) hindurch zum Gutauslauf (44) führbar ist,
- mindestens eine innerhalb des Gehäuses (40) angeordnete Elektronenquelle (20) zum Erzeugen eines Elektronenstrahls,
- eine im Gutführungskanal (41) angeordnete Behandlungszone (19), in der das Gut insbesondere frei fallend mittels des Elektronenstrahls pasteurisierbar oder sterilisierbar ist,
wobei die Vorrichtung (10) mindestens eine innerhalb des äusseren Gehäuses (40) angeordnete und den Gutführungskanal (41) insbesondere entlang der Gutführungsrichtung (R) umschliessende innere Abschirmung (51, 52) mit mindestens einem Abschirmelement (54, 55, 56, 57) zum Abschirmen der bei der Behandlung entstehenden Strahlung aufweist,
**dadurch gekennzeichnet, dass**
das Gehäuse (40) mindestens eine Öffnung (60, 61) aufweist, welche mittels mindestens eines ihr zugeordneten Verschlusselements (62, 62', 63, 63'), insbesondere einer Tür (62, 62', 63, 63'), verschliessbar ist und wobei das Verschlusselement (62, 62', 63, 63') an einer Innenseite (64) mindestens eines der Abschirmelemente (55, 57) enthält.

2. Vorrichtung (10) gemäss Anspruch 1,
wobei der Gutführungskanal (41) mindestens einen Kanalabschnitt (45, 46) aufweist, in dem ihn die innere Abschirmung (51, 52) labyrinthartig umschliesst.

3. Vorrichtung (10) gemäss Anspruch 2,
wobei der Gutführungskanal (41) einen Guteinlaufkanal (45) enthält, in dem das Gut vom Guteinlauf (43) zur Behandlungszone (19) führbar ist, und wobei mindestens eine innere Abschirmung (51) den Guteinlaufkanal (45) labyrinthartig umschliesst.

4. Vorrichtung (10) gemäss einem der Ansprüche 2 und 3, wobei der Gutführungskanal (41) einen Gutauslaufkanal (46) enthält, in dem das Gut von der Behandlungszone (19) zum Gutauslauf (44) führbar ist, und wobei mindestens eine innere Abschirmung (52) den Gutauslaufkanal (46) labyrinthartig umschliesst.

5. Vorrichtung (10) gemäss einem der vorangehenden Ansprüche,
wobei mindestens eine Abschirmung (51, 52) ein statisches erstes Abschirmelement (54, 56) enthält und das Abschirmelement (55, 57) des Verschlusselements (62) ein zweites Abschirmelement (55, 57) bildet, wobei das erste Abschirmelement (54, 56) und das zweite Abschirmelement (55, 57) in einer Schliessposition des Verschlusselements (62, 62', 63, 63'), in der es die ihm zugeordnete Öffnung (60, 61) verschliesst, gemeinsam den Gutführungskanal (41) insbesondere labyrinthartig umschliessen.

6. Vorrichtung (10) gemäss einem der vorangehenden Ansprüche,
wobei die Abschirmelemente (55, 57) einen mehrlagigen Aufbau mit mindestens einer inneren Lage aus Blei und zwei äusseren Lagen aus einem anderen Material aufweist, wobei die äusseren Lagen insbesondere aus rostfreiem Stahl oder einem Kunststoff bestehen.

7. Vorrichtung (10) gemäss einem der vorangehenden Ansprüche,
wobei der Gutführungskanal (41) durch Wandungselemente (14, 11, 16) begrenzt ist, von denen mindestens eines und bevorzugt sämtliche beweglich, bevorzugt lösbar direkt oder indirekt mit dem Gehäuse (40) verbunden oder verbindbar sind.

8. Verfahren zum Pasteurisieren oder Sterilisieren von partikelförmigem Gut mit einer Vorrichtung (10) gemäss einem der vorangehenden Ansprüche, enthaltend die folgenden Schritte:
a) Erzeugen eines Elektronenstrahls mittels der Elektronenquelle (20),
b) Pasteurisieren oder Sterilisieren des insbesondere frei fallenden Guts mittels des Elektronenstrahls in der Behandlungszone (19).

9. Verfahren gemäss Anspruch 8,
wobei sich das Gut mit einer Geschwindigkeit durch die Behandlungszone (19) bewegt, die im Bereich von 1 m/s bis 5 m/s, bevorzugt von 2 m/s bis 4 m/s, besonders bevorzugt von 2 m/s bis 3 m/s liegt.

10. Verfahren gemäss einem der Ansprüche 8 und 9,
wobei die Elektronen des Elektronenstrahls eine Energie aufweisen, die im Bereich von 80 keV bis 300 keV, bevorzugt von 140 keV bis 280 keV, besonders bevorzugt von 180 keV bis 260 keV liegt.

11. Verfahren gemäss einem der Ansprüche 8 bis 10,
wobei das Gut dem Elektronenstrahl für eine Behandlungszeit ausgesetzt wird, die im Bereich von 5 ms bis 25 ms liegt.

12. Verfahren gemäss einem der Ansprüche 8 bis 11,
wobei das Gut mittels des Elektronenstrahls einer Strahlendosis ausgesetzt wird, die im Bereich von 1 kGy bis 45 kGy, bevorzugt von 8 kGy bis 30 kGy, besonders bevorzugt von 10 kGy bis 16 kGy liegt.

13. Verfahren gemäss einem der Ansprüche 8 bis 12,
der Elektronenstrahl in der Behandlungszone (19) eine mittlere Stromdichte aufweist, die im Bereich von 10¹⁵ s⁻¹·cm⁻² bis 2,77·10¹⁵ s⁻¹·cm⁻² liegt.

14. Verfahren gemäss einem der Ansprüche 8 bis 13,
wobei das Gut aus der Gruppe ausgewählt ist, die besteht aus:
- Lebensmitteln, wie beispielsweise Getreide wie etwa Soja, Frühstückscerealien, Snacks, Nüssen wie etwa getrockneten Kokosnüssen, Mandeln, Erdnussbutter, Kakaobohnen, Schokolade, Schokoladenflüssigkeit, Schokoladenpulver, Schokoladenchips, Kakaoprodukten, Hülsenfrüchten, Kaffee, Samen wie etwa Kürbissamen, Gewürzen (wie beispielsweise Kurkuma, insbesondere in Scheiben), Teemischungen, getrockneten Früchten, Pistazien, trockenen Proteinprodukten, Bäckereiprodukten, Zucker, Kartoffelprodukten, Teigwaren, Babynahrung, getrockneten Eiprodukten, Sojaprodukten wie etwa Sojabohnen, Verdickungsmitteln, Hefen, Hefeextrakten, Gelatine oder Enzymen;
- Tiernahrungsmitteln, wie beispielsweise Pellets, Futter für Wiederkäuer, Geflügel, Wassertiere (insbesondere Fische) oder Haustiere, oder Mischfutter;
- Kunststoff wie etwa PET, beispielsweise in Form von Flocken oder Pellets.

## Claims

1. An apparatus (10) for pasteurizing or sterilizing particulate material, comprising
- an outer casing (40),
- a material inlet (43),
- a material outlet (44),
- a material guide channel (41) in which the material can be guided from the material inlet (43) along a material guide direction (R) through the apparatus (10) to the material outlet (44),
- at least one electron source (20) arranged within the housing (40) for generating an electron beam,
- a treatment zone (19) arranged in the material guide channel (41) in which the material can be pasteurized and/or sterilized, in particular in a free-falling manner, by means of the electron beam,
wherein the apparatus (10) has at least one inner shielding (51, 52) arranged inside the outer housing (40) and enclosing the material guide channel (41), in particular along the material guide direction (R), with at least one shielding element (54, 55, 56, 57) for shielding the radiation produced during the treatment,
**characterized in that**
the housing (40) has at least one opening (60, 61) which can be closed by means of at least one closure element (62, 62', 63, 63') associated therewith, in particular a door (62, 62', 63, 63'), and wherein the closure element (62, 62', 63, 63') contains at least one of the shielding elements (55, 57) on an inner side (64).

2. The apparatus (10) according to claim 1,
wherein the material guiding channel (41) has at least one channel section (45, 46) in which the inner shielding (51, 52) encloses it in a labyrinth-like manner.

3. The apparatus (10) according to claim 2,
wherein the material guide channel (41) contains a material inlet channel (45) in which the material can be guided from the material inlet (43) to the treatment zone (19), and wherein at least one inner shielding (51) encloses the material inlet channel (45) in a labyrinth-like manner.

4. The apparatus (10) according to one of claims 2 and 3, wherein the material guide channel (41) contains a material outlet channel (46) in which the material can be guided from the treatment zone (19) to the material outlet (44), and wherein at least one inner shielding (52) encloses the material outlet channel (46) in a labyrinth-like manner.

5. The apparatus (10) according to any of the preceding claims,
wherein at least one shielding (51, 52) includes a static first shielding element (54, 56) and the shielding element (55, 57) of the closure member (62) forms a second shielding element (55, 57), wherein the first shielding element (54, 56) and the second shielding element (55, 57), in a closed position of the closure element (62, 62', 63, 63'), in which it closes the opening (60, 61) associated with it, together enclose the material guide channel (41), in particular in a labyrinth-like manner.

6. The apparatus (10) according to one of the preceding claims,
wherein the shielding elements (55, 57) have a multilayer structure with at least one inner layer of lead and two outer layers of another material, the outer layers consisting in particular of stainless steel or a plastic.

7. The apparatus (10) according to one of the preceding claims,
wherein the material guide channel (41) is delimited by wall elements (14, 11, 16) of which at least one and preferably all are connected or movably connectable, preferably releasably directly or indirectly to the housing (40).

8. A method for pasteurizing and/or sterilizing particulate material with an apparatus (10) according to one of the preceding claims, comprising the following steps:
a) generating an electron beam by means of the electron source (20),
b) pasteurizing and/or sterilizing the material, in particular freely falling material, by means of the electron beam in the treatment zone (19).

9. The process according to claim 9,
wherein the material moves through the treatment zone (19) at a velocity which is in the range from 1 m/s to 5 m/s, preferably from 2 m/s to 4 m/s, particularly preferably from 2 m/s to 3 m/s.

10. The method according to one of claims 9 and 10,
wherein the electrons of the electron beam have an energy which is in the range from 80 keV to 300 keV, preferably from 140 keV to 280 keV, particularly preferably from 180 keV to 260 keV.

11. The method according to any one of claims 9 to 11,
wherein the material is exposed to the electron beam for a treatment time ranging from 5 ms to 25 ms.

12. The method according to any one of claims 9 to 12,
wherein the material is exposed by means of the electron beam to a radiation dose which is in the range from 1 kGy to 45 kGy, preferably from 8 kGy to 30 kGy, particularly preferably from 10 kGy to 16 kGy.

13. The method according to one of claims 9 to 13,
wherein the electron beam in the treatment zone (19) has an average current density which is in the range from 10¹⁵ s⁻¹·cm⁻² to 2.77 10¹⁵ s⁻¹·cm⁻².

14. The method according to one of claims 9 to 14,
where the material is selected from the group consisting of:
- foods such as cereals such as soybeans, breakfast cereals, snacks, nuts such as dried coconuts, almonds, peanut butter, cocoa beans, chocolate, chocolate liquid, chocolate powder, chocolate chips, cocoa products, legumes, coffee, seeds such as pumpkin seeds, spices (such as turmeric, especially in slices), tea mixtures, dried fruits, pistachios, dried protein products, bakery products, sugar, potato products, pasta, baby food, dried egg products, soy products such as soybeans, thickeners, yeasts, yeast extracts, gelatine or enzymes;
- pet food, such as pellets, feed for ruminants, poultry, aquatic animals (especially fish) or pets, or compound feed;
- plastic such as PET, for example in the form of flakes or pellets.

## Revendications

1. Dispositif (10) pour la pasteurisation ou la stérilisation de produits en forme de particules, comprenant
- un boîtier extérieur (40),
- une entrée de produit (43),
- une sortie de produit (44),
- un canal de guidage de produit (41) dans lequel le produit peut être guidé depuis l'entrée de produit (43) vers la sortie de produit (44) le long d'une direction de guidage de produit (R) à travers le dispositif (10),
- au moins une source d'électrons (20) disposée à l'intérieur du boîtier (40) pour générer un faisceau d'électrons,
- une zone de traitement (19) disposée dans le canal de guidage de produit (41), dans laquelle le produit peut être pasteurisée ou stérilisée, en particulier en chute libre, au moyen du faisceau d'électrons,
le dispositif (10) présentant au moins un blindage intérieur (51, 52) disposé à l'intérieur du boîtier extérieur (40) et entourant le canal de guidage de produit (41), en particulier le long de la direction de guidage de produit (R), avec au moins un élément de blindage (54, 55, 56, 57) pour tamiser le rayonnement produit lors du traitement,
**caractérisé en ce que**
le boîtier (40) présente au moins une ouverture (60, 61) qui peut être fermée au moyen d'au moins un élément de fermeture (62, 62', 63, 63') qui lui est associé, en particulier une porte (62, 62', 63, 63'), et l'élément de fermeture (62, 62', 63, 63') contenant sur un côté intérieur (64) au moins l'un des éléments de blindage (55, 57) .

2. Dispositif (10) selon la revendication 1,
dans lequel le canal de guidage de produit (41) présente au moins une section de canal (45, 46) dans laquelle le blindage interne (51, 52) l'entoure à la manière d'un labyrinthe.

3. Dispositif (10) selon la revendication 2,
dans lequel le canal de guidage de produit (41) contient un canal d'entrée de produit (45) dans lequel le produit peut être guidé de l'entrée de produit (43) vers la zone de traitement (19), et dans lequel au moins un blindage intérieur (51) entoure le canal d'entrée de produit (45) à la manière d'un labyrinthe.

4. Dispositif (10) selon l'une des revendications 2 et 3, dans lequel le canal de guidage de produit (41) comprend un canal de sortie de produit (46) dans lequel le produit peut être guidé de la zone de traitement (19) vers la sortie de produit (44), et dans lequel au moins un blindage intérieur (52) entoure le canal de sortie de produit (46) à la manière d'un labyrinthe.

5. Dispositif (10) selon l'une quelconque des revendications précédentes,
dans lequel au moins un blindage (51, 52) comprend un premier élément de blindage statique (54, 56) et l'élément de blindage (55, 57) de l'élément de fermeture (62) forme un deuxième élément de blindage (55, 57), le premier élément de blindage (54, 56) et le deuxième élément de blindage (55, 57) entourant ensemble, en particulier à la manière d'un labyrinthe, le canal de guidage de produit (41) dans une position de fermeture de l'élément de fermeture (62, 62', 63, 63'), dans laquelle il ferme l'ouverture (60, 61) qui lui est associée.

6. Dispositif (10) selon l'une des revendications précédentes,
dans lequel les éléments de blindage (55, 57) présentent une structure multicouche avec au moins une couche interne en plomb et deux couches externes en un autre matériau, les couches externes étant notamment en acier inoxydable ou en une matière plastique.

7. Dispositif (10) selon l'une des revendications précédentes,
dans lequel le canal de guidage de produit (41) est délimité par des éléments de paroi (14, 11, 16), dont au moins un et de préférence tous sont reliés ou peuvent être reliés de manière mobile, de préférence de manière amovible, directement ou indirectement au boîtier (40).

8. Procédé de pasteurisation ou de stérilisation de produits en forme de particules avec un dispositif (10) selon l'une des revendications précédentes, comprenant les étapes suivantes :
a) générer un faisceau d'électrons au moyen de la source d'électrons (20),
b) la pasteurisation ou la stérilisation du produit, en particulier en chute libre, au moyen du faisceau d'électrons dans la zone de traitement (19).

9. Procédé selon la revendication 8,
dans lequel le produit se déplace à une vitesse qui se situe dans la plage de 1 m/s à 5 m/s, de préférence de 2 m/s à 4 m/s, de manière particulièrement préférée de 2 m/s à 3 m/s.

10. Procédé selon l'une quelconque des revendications 8 et 9, dans lequel les électrons du faisceau d'électrons ont une énergie qui se situe dans la plage de 80 keV à 300 keV, de préférence de 140 keV à 280 keV, de manière particulièrement préférée de 180 keV à 260 keV.

11. Procédé selon l'une quelconque des revendications 8 à 10, dans lequel le produit est exposé au faisceau d'électrons pendant une durée de traitement comprise entre 5 ms et 25 ms.

12. Procédé selon l'une quelconque des revendications 8 à 11, dans lequel le produit est exposé à une dose de rayonnement qui se situe dans la plage de 1 kGy à 45 kGy, de préférence de 8 kGy à 30 kGy, de manière particulièrement préférée de 10 kGy à 16 kGy.

13. Procédé selon l'une quelconque des revendications 8 à 12, dans lequel le faisceau d'électrons dans la zone de traitement (19) présente une densité de courant moyenne qui se situe dans la plage de 10¹⁵ s⁻¹·cm⁻² à 2,77·10¹⁵ s⁻¹ cm⁻².

14. Procédé selon l'une quelconque des revendications 8 à 13, dans lequel le produit est choisie dans le groupe constitué par :
- des aliments tels que les céréales comme le soja, les céréales pour petit-déjeuner, les snacks, les noix comme les noix de coco séchées, les amandes, le beurre de cacahuètes, les fèves de cacao, le chocolat, le chocolat liquide, le chocolat en poudre, les pépites de chocolat, les produits à base de cacao, les légumineuses, le café, les graines comme les graines de citrouille, des épices (telles que le curcuma, en particulier en tranches), des mélanges de thé, des fruits séchés, des pistaches, des produits protéinés secs, des produits de boulangerie, du sucre, des produits à base de pommes de terre, des pâtes, des aliments pour bébés, des ovoproduits séchés, des produits à base de soja tels que les graines de soja, des épaississants, des levures, des extraits de levure, de la gélatine ou des enzymes ;
- des aliments pour animaux, tels que des granulés, des aliments pour ruminants, volailles, animaux aquatiques (en particulier poissons) ou animaux domestiques, ou des aliments composés ;
- plastique tel que le PET, par exemple sous forme de flocons ou de granulés.
